# EUROPEAN PATENT SPECIFICATION

(11) **EP 2 013 366 B1**
(45) Date of publication and mention of the grant of the patent: **30.11.2016**
(21) Application number: 07797310.5
(22) Date of filing: 01.05.2007
(51) Int. Cl.: C12Q 1/68

(54) **Sequencing of the L10 codon of the HIV gag gene**
Sequenzierung vom L10-Codon vom HIV-gag-Gen
Séquençage du codon L10 du gène gag du VIH

(30) Priority: 01.05.2006 US 746124 P
(43) Date of publication of application: 14.01.2009
(73) Proprietor: Siemens Healthcare Diagnostics Inc., Tarrytown, NY 10591 (US)
(72) Inventor: REYNOLDS, Rebecca, Milford, MA 01757 (US)
(74) Representative: Schweitzer, Klaus
(86) International application number: PCT/US2007/067920
(87) International publication number: WO 2007/130967

(56) References cited:
- WO-A-01/98539
- WO-A-03/025202
- US-A- 5 866 336
- US-A1- 2002 028 455
- US-A1- 2002 160 361
- US-A1- 2003 219 770
- ROUDINSKII NIKITA I ET AL: "Diversity of human immunodeficiency virus type 1 subtype A and CRF03-AB protease in eastern Europe: Selection of the V77I variant and its rapid spread in injecting drug user populations" JOURNAL OF VIROLOGY, vol. 78, no. 20, October 2004 (2004-10), pages 11276-11287, XP002549418 ISSN: 0022-538X
- WILTON ET AL. HUMAN MUTATION vol. 11, 1998, pages 252 - 258, XP002094957
- Robert W Shafer ET AL: "A Guide to HIV-1 Reverse Transcriptase and Protease Sequencing for Drug Resistance Studies", HIV sequence compendium, 1 January 2001 (2001-01-01), pages 1-51, XP055254236, Retrieved from the Internet: URL:http://www.ncbi.nlm.nih.gov/pmc/articl es/PMC3274565/pdf/nihms65128.pdf

## Description

### FIELD OF INVENTION

The present invention relates to the field of DNA synthesis using nucleic acid primers.

### BACKGROUND OF INVENTION

Nucleic acid primers are commonly used to initiate *in vitro* DNA replication for polymerase-catalyzed nucleic acid sequencing, polymerase chain reaction amplification, and transcription and reverse-transcription of nucleic acid templates. The nucleic acid primer (a short polynucleotide) is complementary to and hybridizes to a target region of a polynucleotide template, and, in the presence of a nucleic acid polymerase enzyme and nucleic acid building blocks (deoxyribonucleoside triphosphates, dATP, dCTP, dGTP, dTTP, dUTP, and modified nucleotides), initiates synthesis of a new polynucleotide strand that is complementary to the original polynucleotide template.

The presence of polymorphisms directly under or proximate to the primer site, however, often precludes the use of template-specific primers that would hybridize to and prime synthesis of only one of the polymorphic variants. The problem of polymorphic variation directly under a primer is often solved by using degenerate primers, a mixture of different primers having specificity for each polymorphic variant, which hybridize to a "variable" population of primary templates.

Polymorphic variation near the target region of interest presents unique challenges. For example, insertion or deletion mutations located upstream near the start point of the desired sequence of interest may present significant problems if primers must be positioned upstream of these mutations. Resulting amplification products or sequencing fragments will be heterogeneous, containing mixtures of the insertions and/or deletions and resulting in multiple bases from the different sequences at each position downstream of the mixtures that cannot be resolved. Although the sequence of the desired target region may be obtained from the anti-sense strand, the absence of bi-directional confirmatory sequence could compromise critical diagnostic or therapeutic clinical decisions. In such circumstances, primers must therefore be located downstream of the region containing polymorphic variation. In some cases, however, there is not sufficient "lead" sequence between the undesirable polymorphic variation and the start of the region of interest to locate a primer and obtain reliable sequence data at the desired start point. Due to the presence of contaminating artifacts (such as dye blobs) and low resolution and separation of peaks within the initial 30-60 bases of new sequence that can interfere with accurate basecalling, primers must be located at an appropriate distance upstream. If polymorphic variation occurs in this region, the primers must be located closer to or at the desired start point in the target sequence, resulting in a shorter "lead" sequence and possibly a loss of DNA sequence information will be obtained for that region. US2003/219770 A1 is related to sequencing but contains an "abasic" linker between the universal sequence and the target specific sequence which would interfere with the sequencing method to be used.

US2002/160361 A1 and WO03/025202 A also published in the field of gene technology refer to analysis of gene expression and amplification of nucleic acids but not sequencing.

Shafer et al. (HIV sequence compendium, 2001, pages 1-51) discloses a sequencing approach to determine the genotype of the L10 codon in gag to evaluate drug resistance in HIV.

Accordingly, there is a need to improve sequencing accuracy of nucleotide sequences located proximate to insertion/deletion mutations.

### SUMMARY OF INVENTION

The present invention is directed to methods and primers for improving the accuracy of basecalling at the start point of the desired sequence. More specifically, the methods and primers of the present invention improve the accuracy of basecalling of nucleotide sequences located near refractory regions and provide a way to obtain the same sequencing start point from all samples, which minimizes repeat testing and increases overall success rate in the clinical laboratory.

The oligonucleotide forward primers of the invention comprise the sequence of any of SEQ ID NO: 8, 9, 11, 12, 13. The present also specifically relates to a method to prepare a template for sequencing the 10 codon of the HIV protease gag gene comprising the steps of: providing a polynucleotide template having refractory region upstream of a target region to be sequenced; hybridizing to the polynucleotide template an oligonucleotide forward primer of SEQ ID NO: 8, 9, 11, 12 or 13; initiating polymerase-catalyzed DNA synthesis, thereby producing a second replicate polynucleotide template comprising the above oligonucleotide forward primer, for use in a subsequent sequencing reaction.

### DETAILED DESCRIPTION OF PREFERRED EMBODIMENTS

### Definition

While the terminology used in this application is standard within the art, the following definitions of certain terms are provided to assure clarity.

Units, prefixes, and symbols may be denoted in their SI accepted form. Unless otherwise indicated, nucleic acids are written left to right in 5' to 3' orientation. Numeric ranges recited herein are inclusive of the numbers defining the range and include and are supportive of each integer within the defined range. Amino acids may be referred to herein by either their commonly known three letter symbols or by the one-letter symbols recommended by the IUPAC-IUBMB Nomenclature Commission. Nucleotides, likewise, may be referred to by their commonly accepted single-letter codes. Unless otherwise noted, the terms "a" or "an" are to be construed as meaning "at least one of." The section headings used herein are for organizational purposes only and are not to be construed as limiting the subject matter described. In the case of any amino acid or nucleic sequence discrepancy within the application, the figures control.

**"Base"** means a nucleic acid base consisting of any complex compound composed of purines, pyrimidines, carbohydrates, and phosphoric acid. Nucleic acids are commonly in the form of DNA or RNA and its equivalents. The term "base" includes not only nucleic acid bases, but also its corresponding equivalent forms, such as nucleobases.

**"Base-calling"** means a computational process of identifying establishing a sequence of nucleotides in a DNA molecule. The data representing the basecalls are generally in the form of chromatogram, a series of peaks arranged in order of molecular weight according to the size-distribution in a molecular sieving medium.

**"Corresponding"** means having the same or nearly the same relationship with respect to position and complementarity as between two nucleotide sequences, or having the same or nearly the same relationship with respect to structure, function, or genetic coding (for example, as between a gene and the "corresponding" protein encoded by the gene). For example, a nucleotide sequence is said to "correspond" to a region of a polynucleotide template if the two sequences are identical or complementary or have portions that are identical or complementary.

**"DNA synthesis"** means *in vitro* replication of a single-stranded polynucleotide template by hybridization of a complementary oligonucleotide primer to the template, followed by sequential addition to the primer of deoxyribonucleoside triphosphates (dATB, dGTP, dCTP, dTTP, dUTP and modified bases having similar structures, such as inosine, isoC, isoG, etc.) complementary to corresponding nucleotides of the template, in the presence of a DNA polymerase enzyme.

**"Downstream"** means located in the direction of or toward the 3' end of a polynucleotide.

**"Extension sequence"** means a polynucleotide sequence that extends the length of an oligonucleotide primer and/or serves as a spacer sequence between a universal nucleotide sequence and a target-specific oligonucleotide primer sequence. As used herein, the term "extension sequence" expressly excludes universal tag sequences having functionality as universal priming templates. As discussed in more detail below, extension sequences may comprise sequence that is non-complementary to the template, sequence that is complementary to the template, or a combination of non-complementary and complementary sequence.

**"Linking"** and **"linked,"** as used in reference to the extension nucleotide sequence "linking" two sequences or "linked to" another sequence, means that an extension nucleotide sequence is covalently linked to another nucleotide sequence by a phosphodiester bond through the 3'-hydroxyl (-OH) group of one sugar and the 5'-phosphate group of an adjoining sugar, to thereby form a polynucleotide chain "linked" through a polynucleotide sugar-phosphate backbone. Nucleotide sequences that are linked are amenable to polymerase-catalyzed DNA synthesis as a single unit. An extension nucleotide sequence "linking" the universal sequence and the target-specific sequence consists of an extension nucleotide interposed between and connected to the universal sequence and the target-specific sequence by phosphodiester bonds.

**"Nucleic acid"** and **"polynucleotide"** are considered to be equivalent and interchangeable, and refer to polymers of nucleic acid bases comprising any of a group of complex compounds composed of purines, pyrimidines, carbohydrates, and phosphoric acid. Nucleic acids are commonly in the form of DNA or RNA. The term "nucleic acid" includes polynucleotides of genomic DNA or RNA, cDNA, semisynthetic, or synthetic origin. Nucleic acids may also substitute standard nucleotide bases with nucleotide isoform analogs, including, but not limited to iso-C and iso-G bases, which may hybridize more or less permissibly than standard bases, and which will preferentially hybridize with complementary isoform analog bases. Many such isoform bases are described, for example, at www.idtdna.com. The nucleotides adenosine, cytosine, guanine and thymine are represented by their one-letter codes A, C, G, and T respectively. In representations of degenerate primers or mixture of different strands having mutations in one or several positions, the symbol R refers to either G or A, the symbol Y refers to either T/U or C, the symbol M refers to either A or C, the symbol K refers to either G or T/U, the symbol S refers to G or C, the symbol W refers to either A or T/U, the symbol B refers to "not A", the symbol D refers to "not C", the symbol H refers to "not G", the symbol V refers to "not T/U" and the symbol N refers to any nucleotide. It is understood that polynucleotide molecules are typically associated in a double-helix duplex, with one polynucleotide strand acting as the "sense" strand and the other complementary polynucleotide strand acting as the "antisense" strand. Because DNA is generally in the form of a duplex, comprising a sense strand and an antisense strand that is the reverse complement of the sense strand, the sequence of one strand can be inferred from the sequence of the other strand. For purposes of clinical determination of sequence, however, bi-directional sequence (sequence of both strands) is necessary to confirmation accuracy.

**"Oligonucleotide"** means a polynucleotide molecule, generally of shorter length.

**"Polynucleotide template"** means a single-stranded polynucleotide chain that includes a target nucleotide region of interest and which serves as a template or substrate for binding of an oligonucleotide primer and polymerase-catalyzed DNA synthesis in the presence of a DNA polymerase enzyme and deoxyribonucleoside triphosphates building blocks. Although a polynucleotide template will generally consist of a single polynucleotide molecule, it is understood that reference to a "polynucleotide template" also encompasses the corresponding sense or anti-sense strand.

**"Polymerase-catalyzed"** means produced in a reaction catalyzed by a DNA polymerase enzyme.

**"Primer"** means an oligonucleotide molecule having a free 3'-hydroxyl group available for polymerase-catalyzed covalent bonding with a 5'-triphosphate group of a deoxyribonucleoside triphosphate molecule.

**"Proximate,"** as used in reference to the number of base pairs between an insertion/deletion region of a polynucleotide template and the 5' terminus of a target region of the template, means a distance that is too close to enable consistently accurate basecalling due to the presence of contaminating artifacts, and low resolution and separation of peaks. Generally, the first 30-60 basepairs of a nucleotide sequence cannot be accurately determined, due to the presence of such artifacts that interfere with the ability to identify and characterize the signal peak at that location. Insertion/deletion mutations that are greater than 30-60 base pairs from the target region being amplified and/or sequenced will not interfere with sequencing, provided acceptable primers sites are available within the 30-60 base pair region between the mutation region and the desired start point of the sequence of interest.

**"Refractory region"** means a region of a polynucleotide template that is difficult to amplify or sequence. Typically, a region of a polynucleotide template that is difficult to amplify or sequence will result in ambiguous or unreliable sequence results or is comprised of nucleotide sequence for which the design of primers is difficult. A refractory region may be characterized, for example, by insertion or deletion mutations, high variability between individuals and across subtypes, clusters of resistance mutations, nucleotide repeats, and regions that do not conform to standard primer design rules.

**"Region of interest"** means the region of a polynucleotide template for which accurate nucleotide sequence is desired.

**"Replicate,"** as used in reference to "replicates" of an original polynucleotide template, means a complementary copy of the polynucleotide template and subsequent complementary copies thereof.

**"Sequence"** *(noun)* means the order of nucleotide bases in a polynucleotide molecule.

**"Sequence"** *(verb)* or "sequencing" means the chemical or enzymatic process of generating fragments of nucleic acid or polynucleotide molecule in order to determine the order of nucleotides in the molecule. A well known method of sequencing is the "chain termination" method first described by Sanger et al., PNAS (USA) 74(12): 5463-5467 (1977) and detailed in Sequenase® 2.0 product literature and more recently elaborated in European Patent EP-B1-655506. In this process, DNA to be sequenced is isolated, rendered single stranded, and placed into four vessels. In each vessel are the necessary components to replicate the DNA strand, which include a template-dependent DNA polymerase, a short primer molecule complementary to the initiation site of sequencing of the DNA to be sequenced and deoxyribonucleoside triphosphates for each of the bases A, C, G and T, in a buffer conducive to hybridization between the primer and the DNA to be sequenced and chain extension of the hybridized primer. In addition, each vessel contains a small quantity of one type of dideoxynucleoside triphosphate, e.g. dideoxyadenosine triphosphate ("ddA"), dideoxyguanosine triphosphate ("ddG"), dideoxycytosine triphosphate ("ddC"), dideoxythymidine triphosphate ("ddT"). In each vessel, each piece of the isolated DNA is hybridized with a primer. The primers are then extended one base at a time to form a new nucleic acid polymer complementary to the template DNA. When a dideoxynucleotide is incorporated into the extending polymer, the polymer is prevented from further extension. Accordingly, in each vessel, a set of extended polymers of specific lengths are formed which are indicative of the positions of the nucleotide corresponding to the dideoxynucleotide in that vessel. The identity of the terminal nucleotide (as A, C, G or T) can be determined by using either labeled primers (a different label for each of the A, C, G or T) or by using labeled dideoxynucleoside terminator molecules. These sets of polymers are then evaluated using gel electrophoresis to determine the sequence.

Sequencing of polynucleotides may be performed using either single-stranded or double-stranded DNA. Use of polymerase for primer extension requires a single-stranded DNA template. In preferred embodiments, the method of the present invention uses double-stranded DNA and both strands are sequenced to obtain confirmatory sequence from the opposite strand. Double-stranded DNA templates may be prepared for sequencing using either alkaline or heat denaturation to separate the two complementary DNA templates into single strands. During polymerization, each molecule of the DNA template is copied once as the complementary primer-extended strand. Use of thermostable DNA polymerases (e.g. *Taq, Bst, Tth or Vent* DNA polymerase) enables repeated cycling of double-stranded DNA templates in the sequencing reaction through alternate periods of heat denaturation, primer annealing, extension and dideoxy termination. This cycling process effectively amplifies small amounts of input DNA template to generate sufficient template for sequencing.

Sequencing may also be performed directly on PCR amplification reaction products. Although the cloning of amplified DNA is relatively straightforward, direct sequencing of PCR products facilitates and speeds the acquisition of sequence information. As long as the PCR reaction produces a discrete amplified product, it will be amenable to direct sequencing. In contrast to methods where the PCR product is cloned and a single clone is sequenced, the approach in which the sequence of PCR products is analyzed directly is generally unaffected by the comparatively high error rate of Taq DNA polymerase. Errors are likely to be stochastically distributed throughout the molecule and not detected. Thus, the majority of the amplified product will consist of the correct sequence. Direct sequencing of PCR products has the advantage over sequencing cloned PCR products in that (1) it is readily standardized because it is a simple enzymatic process that does not depend on the use of living cells, and (2) only a single sequence needs to be determined for each sample.

**"Sequencing leader"** means that initial portion of the polynucleotide sequencing template with respect to which basecalling is not consistently reliable and accurate. A sequencing leader typically is 30-60 bases from the 3' end of the primer used for sequencing, depending on the sequencing chemistry used. The sequencing leader of a polynucleotide sequencing template will therefore include any extension sequence incorporated by the chimeric primer, and possibly also a portion of the target region sequence.

**"Target-specific sequence,"** as used in reference to a region of the oligonucleotide primer of the present invention, means that region sufficiently complementary to be capable of hybridizing to the corresponding target region of a polynucleotide template that is being amplified and/or sequenced.

**"Target region"** means the region of a polynucleotide template that is being amplified and/or sequenced. It is understood that the term "target region" may be used to refer to the specific region that is sufficiently complementary to be capable of hybridizing the oligonucleotide primer target-specific sequence, or, alternatively, to the entire region of the polynucleotide template that is being amplified and/or sequence (i.e., including the region of the polynucleotide template downstream of the primer region).

**"Universal sequence"** means a nucleotide sequence to which a standard primer specifically hybridizes. Universal sequences are commonly known and used in the art. By way of example, a common universal sequence primer includes a sequence that hybridizes specifically to a region of the M 13 vector near the 5' end of the cloned insert. Specific examples of universal sequencing primers known in the art are -21M13, M 13-40 and -36M13. In the context of the present invention, it is understood that the term "universal sequence" may be used not only in reference to a nucleotide sequence to which a universal primer hybridizes, but also to a complementary sequence that serves as the template for the synthesis of the universal sequence.

**"Upstream"** means located in the direction of or toward the 5' end of a polynucleotide.

The present disclosure relates to novel oligonucleotide primers for initiating polymerase-catalyzed DNA synthesis at a target region on a polynucleotide template, as well as methods of using such oligonucleotide primers and kits containing such oligonucleotide primers. The primers disclosed may be used, for example, for the purpose of facilitating replication, amplification, transcription or reverse-transcription of a template nucleic acid molecule.

More particularly, the present disclosure relates to chimeric oligonucleotide primers that have an extension sequence linked to the 5' end of the primer. When the hybrid oligonucleotide (including the extension sequence) is used as a primer, the resulting new strand of DNA that is generated by polymerase-catalyzed synthesis includes the primer sequence (the 3' target-specific primer sequence corresponding to a target region on the polynucleotide template, as well as the extension sequence) and any downstream sequence that is generated until termination of the new strand. The "copy" of the nucleotide template that is generated thus includes the extension sequence, effectively replacing the upstream sequence of the original template with the extension sequence of the primer. The primers of the present invention are, therefore, advantageous when it is desirable to replace the upstream sequence of the original template with sequence that is more amenable to replication, for example, when the upstream region contains sequence that is itself refractory to sequencing due to the presence of polymorphisms or contains sequence that is not amenable to primer design. The primers of the present disclosure are particularly advantageous when the upstream regions refractory to sequencing are located proximate to the region of interest for which accurate sequence must be obtained and would not produce sequencing templates having sufficient lead sequence to enable accurate sequence results beginning at a particular region of interest.

The particular problem giving rise to the present invention was the inability to obtain reliable sequence data of polynucleotide templates proximate to regions refractory to sequencing. Specifically, the inventors were unable to obtain bidirectional sequence for the L10 codon (a resistance mutation codon) of the HIV protease gene in all samples as a result of an upstream region, located near the region of interest, that was refractory to primer design and/or sequencing. Approximately 5-15% of samples cannot be sequenced in the forward direction because of mixtures of insertion and deletion mutations in a highly polymorphic region of the HIV genome immediately upstream of the protease gene. Although current sequencing strategies utilize an alternate sequencing primer to capture the forward direction, the primer location is constrained to a region within the protease gene very near the start sequence, resulting in sequence for the first 15-20 codons of the protease gene (including the L10 codon) not being determinable when the alternate primer is used. Notwithstanding the availability of this alternative primer, it is inadequate for purposes of clinical HIV testing, which requires bidirectional confirmatory sequence for the L10 codon. Ideally, all of the initial protease gene codons would be sequenced bidirectionally. The problem thus encountered was that regions refractory to sequencing constrained the placement of primers to locations actually within the region of interest, producing a sequencing template that resulted in the sequence data for the region of interest being unobtainable or confounded by sequencing artifacts and irregular or overlapping peaks that typically occur in the initial sequence data.

Another problem resolved by the present invention is the presence of sporadic dye terminator artifact peaks, commonly referred to as "dye blobs", which occur in the first 30-60 bases from the sequencing start point of the polynucleotide template and produce a strong signal that may interfere with accurate basecalling. In accordance with the present invention, universal sequencing tags were incorporated into the two pairs of PCR primers used to generate sequencing template from the HIV genome. In addition, the primers also included an extension sequence inserted between the universal tag sequence and the HIV-specific primer sequence. The particular extension sequence utilized was a non-complementary spacer sequence of 30-40 bases, which extended the length of the primer extension products by a length sufficient to allow reliable forward sequence to be generated starting at codon 7 of the protease gene, regardless of the sequences or mixtures within the upstream polymorphic region. By using a 40-base extension sequence in the PCR primer with an additional ∼20-base sequence complementary to the target, the sequencing template contained ∼60 bases of sequence upstream of the start point of the desired sequence. Since the largest dye blob signals typically occur within the first ∼60 bases of sequence, the dye blobs interfered only with the initial sequence from the primer (which was already known) and not with the sequence corresponding to the region of interest. Combined with the reverse sequence, bidirectional sequence information for protease codon L10 could be obtained, thus satisfying clinical testing criteria requiring both forward and reverse sequence of this important L10 codon.

The strategy employed in the above situation can readily be extended to any analogous situation where a region refractory to amplification or sequencing is located proximate to the region for which accurate sequence data must be obtained. In accordance with the above discovery, the present invention provides novel oligonucleotide primers, and methods for initiating polymerase-catalyzed DNA synthesis at a target region on a polynucleotide template that is proximate to an upstream region that is refractory to sequencing. The methods and products of the present invention further provide uniform sequence data for clinical interpretation software that will not accept data that does not include specified bases and would be rejected due to interference from sequencing artifacts and ambiguities.

### Specific embodiments

In one aspect, the disclosure relates to oligonucleotide primers comprising a 5' universal sequence, a 3' target-specific primer sequence corresponding to a target region on the polynucleotide template, and an extension sequence linking the universal sequence and the target-specific primer sequence.

The present disclosure also relates to methods that utilize the above novel oligonucleotide primers to initiate polymerase-catalyzed DNA synthesis at a target region on a polynucleotide template. In one aspect, the methods comprise the steps of (1) providing a polynucleotide template having refractory region upstream of a target region; (2) hybridizing to the polynucleotide template an oligonucleotide primer comprising a 5' universal sequence, a 3' target-specific primer sequence corresponding to a target region on the polynucleotide template, and an extension sequence linking the universal sequence and the target-specific sequence; and (3) initiating polymerase-catalyzed DNA synthesis, thereby producing a second polynucleotide template replicate comprising a 5' universal sequence, an extension sequence, the target region and a region of interest.

### Target-Specific Sequence

The oligonucleotide primers of the present disclosure include a target-specific sequence. The target-specific primer sequence comprises a portion that will hybridize to all or part of the target region of the template sequence. The target-specific sequence is preferably 100% complementary to the target region, but need only be substantially complementary, such that the target-specific sequence is able to hybridize specifically to the target region of the template. In particular embodiments, the 3' target-specific primer sequence corresponds to a target region on a polynucleotide template having a region upstream of a target region that is refractory to sequencing. The 3' target-specific primer sequences of the oligonucleotide primers of the invention are linked to a 5' extension sequence.

In some embodiments, the 3' target-specific primer sequence corresponds to a target region on a polynucleotide template having a region upstream of a target region that is refractory to sequencing. In these embodiments, the refractory region of the polynucleotide template is located upstream of and proximate to the target region. In particular embodiments, where the polynucleotide template includes a refractory region upstream of the target region, the number of bases comprising the extension nucleotide sequence is greater than the number of bases between the target region and the refractory region.

The present disclosure uses oligonucleotide primers having a target-specific primer sequence and an extension sequence linked to the target-specific primer sequence. The principal advantages of such a primer design are realized when the region of interest is near a region that is refractory to sequencing and the oligonucleotide primer/extension sequence combination is used to circumvent such refractory regions. The problems associated with refractory regions are circumvented because the additional extension sequence of the oligonucleotide primer is incorporated into the new DNA strand generated in the process of polymerase-catalyzed DNA synthesis, thereby effectively replacing the original polynucleotide template upstream of the primer site. The new DNA strand that incorporates the extension sequence then becomes a template in subsequent DNA synthesis reactions. This process is repeated through multiple rounds of amplification, producing additional DNA templates having the new extension sequence, with all replicate strands generated in subsequent polymerase-catalyzed DNA synthesis steps including the new extension sequence. Eventually, the predominant DNA template in the reaction is the template having the new extension sequence. This amplified DNA template (which includes the extension sequence) is then used as the sequencing template.

As will be appreciated by those in the art, the orientation of the two priming sites is different. That is, one PCR primer will directly hybridize to the first priming site, while the other PCR primer will hybridize to the complement of the second priming site. Stated differently, the first priming site is in sense orientation, and the second priming site is in antisense orientation.

### Extension Sequence

The primers of the present disclosure include an extension sequence that is linked to the target-specific sequence. A universal sequence (discussed below) is attached to the 5' end of the extension sequence.

The function of the primer having the extension sequence is to generate a sequencing template that contains approximately 50-65 bases (30-40 non-complementary sequence plus 20-25 bases of complementary sequence) upstream of the target sequence start point.

The extension sequence used in the oligonucleotide primers of the present invention may consist solely of sequence that is non-target-specific sequence (i.e., non-complementary to the target), may be comprised of a combination of non-target-specific sequence and target-specific sequence, or may consist solely of target-specific sequence.

In the case where the extension sequence comprises a combination of non-target-specific sequence and target-specific sequence, the non-target-specific sequence may be interposed between two or more non-contiguous target-specific sequences, whereby hybridization of the oligonucleotide primer to the polynucleotide template forms a loop-out region of the extension sequence. In another embodiment, where the extension nucleotide sequence comprises non-target-specific sequence and target-specific sequence, the target-specific sequence may consist of all of the sequence between the target region and the refractory region, or part of the sequence between the target region and the refractory region.

The extension sequence may also consist solely of target-specific sequence. An extension sequence consisting of only target-specific sequence may span a refractory region and consequently contain multiple regions that are complementary to some but not all HIV subtypes. The ∼60 bases of the combined target-specific extension sequence and target-specific 3' end of the primer can hybridize sufficiently to the polynucleotide template, even in the presence of multiple mismatches and deletions/insertions that result in loop-outs, to produce PCR products that in turn serve as template in subsequent rounds of amplification. Alternatively, the extension sequence may consist of multiple regions that are complementary to corresponding non-contiguous regions of the template, which may result intervening regions of the polynucleotide template not complementary to the extension sequence looping out. After multiple rounds of amplification, the predominant DNA product in the reaction will have the extension sequence contained in the primer, regardless of the original sequence in the refractory sequence. In addition, samples containing mixtures of insertions and deletions will now generate just one sequence during the sequencing reaction.

It is understood that any target-specific sequence in the primers of the present invention will generally correspond to sequence of one or more (but not necessarily all) regions upstream of the target region, up to and including the refractory region. In addition, the target-specific sequence of the primers will be specific to the desired target so as to avoid non-specific binding of the primer to other regions.

The length of the extension sequence will vary according to circumstances. The length of the extension sequence is determined in part by the dye chemistry (primer vs. terminator) and the particular dye terminator/sequencer combination being used. There will generally be a practical upper limit to the length of the extension sequence that results in inefficient incorporation of the primer into PCR product. The minimum length of the extension sequence will depend on the location of the region of interest relative to regions upstream that are refractory to sequencing, and the location of acceptable primer sites in the intervening region. Where the primers of the present invention are used to circumvent the effects of a refractory region proximate to the region of interest, the extension sequence will generally be of sufficient length to generate a replicate of the polynucleotide template having a sequencing leader (the portion of the nucleotide sequence that is upstream of the region of interest and for which sequence data is not necessary) of sufficient length to enable accurate sequencing of the region of interest. For example, where the refractory region is within only a few bases of an acceptable primer site and a universal sequencing tag is used, the extension sequence will have approximately 40-50 bases which, in addition to the 18-25 bases of the target-specific region of the primer, would generate a sufficiently long sequencing leader to permit accurate determination of the sequence beginning at the region of interest. Where, on the other hand, the refractory region is located a greater distance upstream from an acceptable primer site, the extension sequence may be shorter, depending on the location of an acceptable primer site. A target region consisting of 20 bases that is located approximately 20 bases upstream from the region of interest (for a total of 40 bases) may, for example, require a an extension sequence of only 10-20 bases in order to generate a replicate having a sequencing leader of 50-60 bases from the sequencing start point. The present disclosure thus contemplates that the extension sequence may consist of any number of bases. The extension sequence may, for example, consist of at least 1 base, at least 5 bases, at least 10 bases, at least 15 bases, at least 20 bases, at least 25 bases, at least 30 bases, at least 35 bases, at least 40 bases, at least 45 bases, at least 50 bases, at least 55 bases, at least 60 bases, or at least 65 bases. The extension may also consist of greater than 70 or more bases.

In some embodiments where the primers are used for the purpose of generating sequencing templates, the length of the extension sequence is selected such that the primer initiates synthesis of replicates comprising a sequencing leader of at least 25 bases. In other embodiments, the sequencing leader may consist of at least 30 bases. In other embodiments, the sequencing leader may consist of at least 35 bases. In yet other embodiments, the sequencing leader may consist of at least 40 bases.

### Universal Sequence

The oligonucleotide primers of the present disclosure include a universal primer sequence at the 5' terminus of the primer. Methods for adding non-complementary sequences to the 5' ends of PCR primers to incorporate a universal sequencing primer sequence have been previously described by McBride, et al. (Clinical Chemistry, 35, 1989, 2196-2201, Automated DNA Sequencing Methods Involving Polymerase Chain Reaction) and are well-known in the art, particularly in the field of dye primer chemistry. For example, the non-complementary primer tags may be derived from M13 sequence, which are commercially available and facilitate direct sequencing of fragments cloned into M13 vectors. M13 sequences are also useful as universal sequencing primers for use in dye terminator sequencing chemistry.

The present disclosure provides primer sets of forward primers that comprise universal sequencing primer sequences. Universal primer sequences are generally chosen to be as unique as possible given the particular assays and host genomes to ensure specificity of the amplification and sequencing. In general, universal primer sequences range in size from about 15 to about 35 based pairs, preferably from about 18 to about 25 based pairs.

The oligonucleotide primers of the present disclosure comprise a 3' target-specific primer sequence corresponding to a target region on the polynucleotide template, an extension sequence linked to the 5' end of the target-specific sequence, and a universal sequence linked to the 5' end of the extension sequence.

### Uses of Primers with Extension Sequence

The primers of the present invention are useful in amplification and sequencing of nucleic acid analytes of interest. In particular, the primers of the present invention are useful in applications where a nucleic acid template is replicated for purposes of analysis or sequencing.

The nucleic acid templates used in the present invention will generally be derived from a biological sample that is the subject of research or clinical analysis. For purposes of analysis, such as sequencing, nucleic acid sequences of interest will first be isolated from a biological sample and amplified according to methods well-known to those skilled in the art.

Analysis of mRNA transcripts and RNA genomes will also generally require that the RNA template be converted to a DNA template using reverse transcription (TR) methods prior to polymerase chain reaction (PCR) amplification, which are well known in the art. Nucleic acid analytes are known to be present in biological samples in variable amounts and quality, depending on the organisms or individual from whom the sample is obtained, the tissue source in which the analyte is present, and the particular methodologies used to isolate and amplify the analyte. Starting material obtained from different individuals usually varies in tissue mass or cell number, RNA integrity or quantity, or experimental treatment. Consequently, standardized isolation and amplification methods do not generally produce nucleic acid starting materials to a relatively constant level. Accordingly, on account of variable amounts of starting cDNA between different patients and specimen samples, as well as variable efficiency of the PCR, it is desirable to normalize the population of genomic DNA sequence to a relatively constant level, thereby permitting standardized methods to be subsequently used, regardless of variability in amounts and quality of the starting nucleic acid samples obtained from different sources. Various normalization methods are well-known to those skilled in the art, which are discussed, for example, by Huggett et al, Genes and Immunity, 1-6 (2005). For clinical applications, it is particularly desirable that the PCR amplification products be normalized prior to subsequent analytical steps such as sequencing. For example, a commercially available kit for sequencing clinical samples, ViroSeq(TM) System (Celera Diagnostics), describes a procedure for normalizing nucleic acid templates prior to the sequencing step.

### Methods of DNA Replication

The present disclosure is also directed to methods for initiating polymerase-catalyzed DNA synthesis at a target region on a polynucleotide template. In one aspect the methods comprise the steps of:
providing a polynucleotide template having a refractory region upstream of a target region; hybridizing to the polynucleotide template an oligonucleotide primer comprising a 3' target-specific primer sequence corresponding to a target region on the polynucleotide template, and an extension sequence linking the universal sequence and the target-specific sequence; and
initiating polymerase-catalyzed DNA synthesis, thereby producing a second polynucleotide template replicate comprising a 5' universal sequence, an extension sequence, the target region and a region of interest.

The function of the additional sequence is to increase the distance between the sequencing start point (first incorporated base after the 3' end of the sequencing primer) and the beginning of the target sequence of interest (which is defined by the base following the 3' end of the target-specific primer). Typically the sequence derived from the first approximately 50-60 bases of the template is of poor quality.

Also, with dye terminator chemistry, dye artifacts ("dye blobs") occasionally interfere with basecalling in this same region. Therefore, by inserting an additional approximately 30-40 bases between the universal sequencing tag and the target-specific complementary sequence, high quality sequencing results can be obtained from the bases immediately adjacent to the 3' end of the target-specific primer. This strategy can be used with both dye terminator and dye primer sequencing chemistries.

This design is particularly valuable when genome-specific features limit the options for primer binding locations. One example of this type of limitation is in the HIV genome near the beginning of the protease gene. Ideally the entire sequence of the protease gene would be generated in both directions for a genotyping assay. To obtain unambiguous sequence from the first protease codon in the forward direction, the primer would need to be located approximately 50 bases upstream. However, this region of the HIV genome is highly polymorphic (high concentration of sequence variation between and within subtypes; contains mixtures of sequences containing different numbers of codons in ∼5-15% of individuals). Consequently, two sets of forward sequencing primers had to be developed for the commercial HIV genotyping kits (TRUGENE and ViroSeq). However, the alternate sequencing primers are located within the first part of the protease gene (TRUGENE primer binds to the sequence for the first six codons), preventing bidirectional sequencing information from this region. One of the codons that cannot be sequenced in both directions when these primers are used is L10. The L10 codon is a known resistance mutation codon. It would be a key assay differentiating factor if we could obtain bidirectional sequence for this codon. By using the 3-part primer (universal tag plus non-complementary sequence plus complementary target sequence) bidirectional sequence can be obtained starting at codon 7.

This primer feature has greatest value when the possible primer binding regions are limited by unalterable aspects of the target sequence, as described above with HIV. However, it can provide a significant benefit when incorporated into any sequencing assay design, particularly those assays for which it is desirable or necessary to be able to obtain uniform sequence information from all samples. For example, clinical interpretation software requires that a specific region of the genome be provided to generate a reportable result. By using the 3-part primer design, the impact of sporadic dye blob sequencing artifacts that interfere with basecalling can be eliminated so that sequence from the same region of the genome will be obtained reliably.

### EXAMPLES

### Example 1 - Design of Universal Extension Primer to Sequence L10 Codon of HIV Protease Gene

The methods and primers of the present invention are illustrated in the following example. The objective of the experiments described below was to develop a sequencing-based HIV genotyping assay that could generate bidirectional sequence for codon L10 of the HIV protease gag gene in all samples. The L10 codon is located downstream (within approximately 30 bases) of a highly polymorphic region characterized by mixed insertion/deletion mutations.

Conventional primer design rules and programs place primer sequences located either upstream or downstream of this variable region. Because approximately 5-15% of patients are infected with multiple quasi-species of HIV, one with the gag ins/del mutation and one without, use of upstream primers results in synthesis of a mixture of sequencing templates that cannot be interpreted beyond the point of polymorphism. In order to avoid this problem, existing genotyping assays utilize an alternate forward primer located downstream of the polymorphic region. However, due to the proximity of the L10 codon to the polymorphic region, alternate placement of primers downstream of the gag insertion/deletion polymorphism places primers only 9 base pairs upstream of the L10 region. Due to the presence of contaminating artifacts, as well as irregular peak shape and spacing, within the first approximately 50 bases from the 3' end of the sequencing primer, basecalling accuracy is compromised in the initial sequence results. The proximity of the alternate primer to the L10 region to the primer therefore prevents sequence results for the first approximately 15-20 protease codons in the initial sequence, including the L10 codon. Although sequence for this region can be obtained from sequence of the reverse strand, bidirectional sequence of codon 10, which is essential for clinical diagnostic purposes, cannot be obtained.

In accordance with the principles of the present invention, the above problem of genotyping the HIV gag protease L10 codon was addressed by designing and testing primers having a universal tag at the 5' end, identical to a universal forward or reverse sequencing primer, followed by a region of non-specific sequence either 30 or 40 bases in length, followed by a HIV-specific region. The sequences of the universal tags, derived from M13 sequence, and the non-specific spacer sequences that were used to construct the primers are listed in the following Table 1. The M13-40 universal tag sequence is published and primers complementary to this sequence are commercially available. The M13007 sequence was designed in-house using the published M13 genome sequence.

The full primer sequences are listed in Table 2 for two regions of the HIV genome (protease and a portion of the RT gene). In Table 2, HIV-specific sequences are in bold type. "F" indicates forward direction and "R" indicates reverse direction. Two overlapping regions of the HIV genome were amplified with these primers. The regions are designated "1" and "2".

**TABLE 1. Sequences of M13 universal tags and non-specific spacer regions.**

| FUNCTION/NAME | **SEQUENCE (5'to 3')** | BASES |
|---|---|---|
| Universal M13 sequence tag/M13 -40 | GTTTTCCCAGTCACGACGTTGTA (SEQ NO ID: 1) | 23 |
| Universal M13 sequence tag/M13007 | TTCTGGCGTACCGTTCCTGTC (SEQ NO ID:2) | 21 |
| Spacer/15 bases_1 | GTTGAGCTGAGAACT (SEQ NO ID:3) | 15 |
| Spacer/30 bases_1 | GTTGAGCTGAGAACTCGAAGACAAGAGCTG (SEQ NO ID: 4) | 30 |
| Spacer/40 bases_1 | | 40 |
| Spacer/40 bases_2 | | 40 |

**TABLE 2. Sequences of primers designed and tested for HIV genotyping assay.**

| **PRIMER NAME** | F/R | REGION | **SEQUENCE (5'- 3')** | M13 | SPACER |
|---|---|---|---|---|---|
| P2Fa | **F** | 1 | TTCCCTCAGATCACTCTTTGG (SEO NO ID:7) | none | none |
| RR058 | **F** | 1 | | -40 | 30 bases_1 |
| RR059 | **F** | 1 | | -40 | 15 bases_1 |
| RR060 | **F** | 1 | | -40 | none |
| RR075 | **F** | 1 | | 007 | 30 bases_1 |
| RR076 | F | 1 | | 007 | 40 bases_1 |
| RR091 | **F** | 1 | | 007 | 40 bases_1 |
| RR054 | **R** | 1 | GAATATTGCTGGTGATCCTTTCCA (SEQ NO ID: 14) | none | none |
| RR061 | **R** | 1 | | -40 | 30 bases_1 |
| RR062 | **R** | 1 | | -40 | 15 bases_1 |
| RR063 | **R** | 1 | | -40 | none |
| RR064 | **R** | 1 | | 007 | 30 bases_1 |
| RR065 | **R** | 1 | | 007 | 15 bases_1 |
| RR066 | **R** | 1 | | 007 | none |
| RR077 | **R** | 1 | | -40 | 30 bases_1 |
| RR078 | **R** | 1 | | -40 | 40 bases_2 |
| RR083 | **F** | 2 | GCCTGAAAATCCATACAATACTCCA (SEQ NO ID:23) | none | none |
| RR086 | **F** | 2 | | 007 | 30 bases_1 |
| RR088 | **F** | 2 | | 007 | 40 bases_1 |
| RR109 | **F** | 2 | | 007 | 30 bases_1 |
| RR085 | **R** | 2 | CAAACTCCCATTCAGGAATCCA (SEQ NO ID:27) | none | none |
| RR087 | **R** | 2 | | -40 | 30 bases_1 |
| RR089 | **R** | 2 | | -40 | 40 bases_2 |
| **RR090** | **R** | 2 | | -40 | 40 bases_2 |

The primers shown in Table 2 comprise (1) a "universal" M13 sequence at the 5' end of the primer, with the forward primer and reverse primer having different M13 sequences (sequence not shown in Table 2, but shown above in Table 1), (2) an extension sequence of 30-40 nucleotides of non-HIV sequence linked to the 3' end of the universal sequence (normal type), and (3) HIV-specific sequences linked to the 3' end of the extension sequence and forming the 3' end of the primer (bold type). The above primers were designed with the objective of being used to generate a sequencing template that incorporated additional 30-40 bases of sequence (a non-specific extension sequence) that would serve to increase the length of the initial sequencing template. The M13-derived sequences were added to facilitate standardized sequencing protocols using M-13 sequencing primers. The final 20-25 bases of the primers are HIV-specific sequence. By effectively adding an additional ∼50-60 bases upstream of the region of interest (i.e., the L10 codon), accurate basecalling could be begin at the region of interest.

To address the effect of a long stretch of non-complementary sequence at the 5' end of the primers, a plasmid containing the HIV genome was amplified with various pairs of primers. P2Fa and RR054 do not contain a universal tag or spacer region and served as a control. Primers containing these sequences and different M13 universal tags with different lengths of spacer sequences (see Table 2) were tested and the yields of PCR products were compared to the product generated with the control pair. Primers with the universal tag and a 30-base spacer sequence could be efficiently incorporated by using an annealing temperature of 55°C for 1 minute for all cycles.

Primers were also designed having different sequences in the spacer region. Sequences were selected that were non-complementary to the HIV genome and its complement, and that had little or no potential of forming secondary structures that would interfere with the initial sequence from the template. A spacer sequence was specifically selected that would result in the new template strand having a relatively high percentage of C and T bases, which would be expected to have less tendency to form secondary structures.

Although the extension regions of the primers for forward and reverse strands were initially identical, with no apparent negative impact on amplification or sequencing efficiency, different sequences for the 40-base extension regions (40 bases_1 and 40 bases_2) were also used for the final forward and reverse primers.

### Example 2 - Length of Spacer Region

The initial assay development studies were performed with primers RR058 and RR064 which each contain the 30-base spacer region. The original sample was a plasmid containing the HIV genome (pHIV-gpt obtained from the NIH AIDS Research & Reference Reagent Program). One of the dye terminator artifacts ("dye blob") was found to overlap the region of the protease codon 10 sequence. Although these artifacts appear sporadically and are independent of operator and clean-up procedures, the location of such artifacts is expected to vary, depending on the particular chemistry and methodology used to generate sequencing fragments. In this particular case, in order to ensure that sequencing runs would not have to be repeated due to the presence of dye blobs, the length of the primer spacer region was extended to 40 bases (RR076 and RR078). In these experiments, the codon 10 sequence was unaffected by the presence of the dye blobs.

### Example 3 - Generation of Sequencing Templates

The primers described above in Table 2 were tested with RT-PCR products obtained from cultured HIV virus particles (ACCUTYPE samples from BBI) and clinical samples. RT-PCR products were prepared from ACCUTYPE samples that had been diluted with basepool from 106 to 250 copies per mL prior to extraction. Multiple samples of HIV subtypes A, B, C, G, and CRF02AG were tested at all dilutions. Agarose gel analysis of sequencing templates generated with RR076 and RR078 showed that PCR products amplified with RR076 and RR078 were 904 bp.

Three different ACCUTYPE samples of subtypes D, F, and CRF01AE were also tested at 5000 copies per mL and the resulting sequencing templates generated with RR076 and RR078 were analyzed by agarose gel electrophoresis.

The RR076 and RR078 primers also were evaluated using RT-PCR products obtained from an in-house panel of 23 clinical samples. The eight HIV subtypes covered by the ACCUTYPE samples above were represented in this panel of clinical samples. The viral loads ranged from 3,000 to 800,000 copies/mL.

Sequencing templates were also generated from an additional region of HIV and primers were synthesized with and without the universal sequencing tags and the 30- and 40-base spacer regions (primers RR083, RR085-RR090, and RR109) for testing on plasmids, ACCUTYPE viral particles, and patient samples. Primer pair RR086 and RR087 was used to generate sequencing template from the same set of ACCUTYPE samples and dilutions. The size of the PCR product sequencing template generated by RR086 and RR087 is 1201 bp.

The same panel of 23 clinical samples was amplified with RR086 and RR087 (1201 bp) and RR109 and RR087 (996 bp). As in the previous experiments, the expected products were produced from all but one of the samples (data not shown).

### Example 4

The RR076 and RR078 primers represent forward and reverse extension primers encompassing a region extending from nucleotides 1817-2551 of HIV (NC001802), and produce a 904 base pair product. The RR086 and RR087 primers represent forward and reverse extension primers for region 2 encompassing a region extending from nucleotides 2273-3322 (NC_001802), and produce a 1201 base pair product.

The base content of the spacer sequence was intentionally biased so that the spacer region in the template would contain a higher percentage of C and T nucleotides. The higher the percentage of C and T nucleotides, the less the secondary structure. Secondary structure can cause the sequencing enzyme to stop so it was desirable to minimize the impact of the spacer region on the efficiency of the sequencing enzyme. A BLAST search of these spacer sequences with the HIV genome did not reveal significant similarities so these sequences are not expected to hybridize with the target.

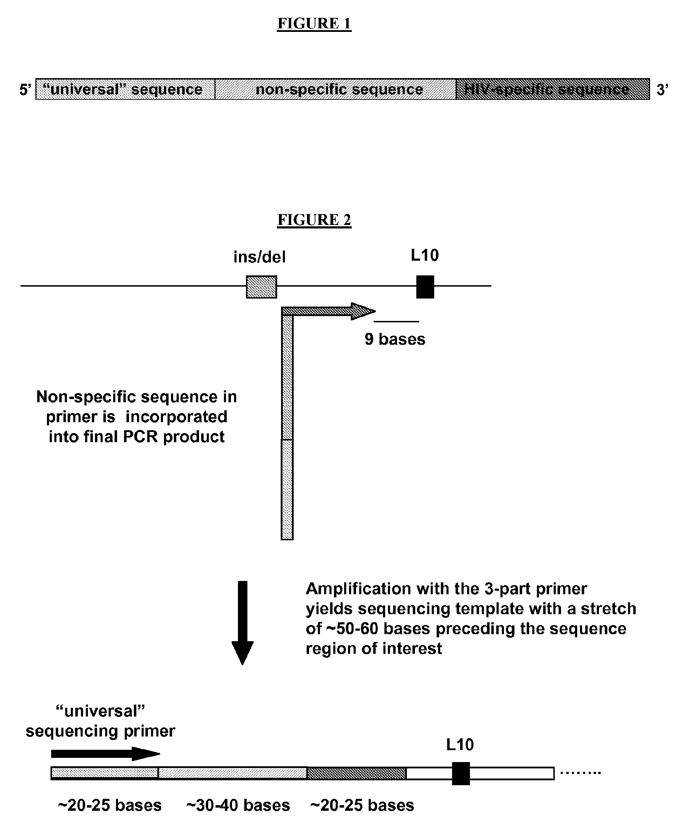

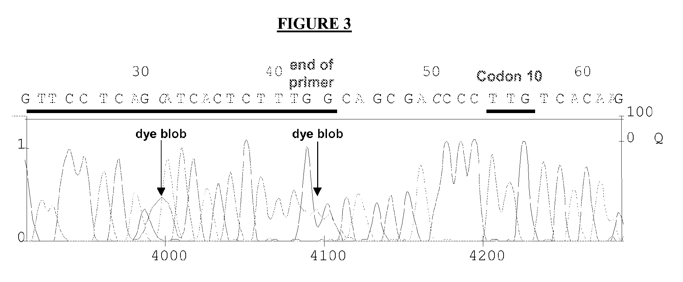

RT-PCR Amplification. The H1V-specific sequence of the chimeric primers were capable of hybridizing to the HIV gag region between the ins/del polymorphic region and the L10 region, as illustrated in FIG. 2, and priming synthesis of a new DNA template for use in sequencing codons 7-99 of the HIV protease gene and codons 1-340 of the HIV RT gene. The same protocols can also be used with a different set of primers to expand the sequenced region of protease to include codons 1-99. The new sequencing templates produced by PCR amplification of this region therefore included the 5' universal sequence, as well as the non-specific extension sequence, linked to the HIV region being amplified. The non-specific extension sequence of the sequencing template thus provides a longer sequence leader upstream of the start point of the region of interest.

Normalized DNA sequencing templates were first prepared by generating PCR product from HIV-gpt plasmid using the QIAGEN HotStarTaq Master Mix Kit. Primers were added to each of the separate PCR kit reagents. Master Mixes were prepared and 45 uL aliquoted into the bottom of each 0.2 mL PCR tube. Five microliters of the template was added per PCR tube. PCR tubes were placed on a thermal cycler prewarmed to 94°C. Thermal cycling conditions were the same for each kit, as follows:

| | |
|---|---|
| 1X | 2' at 94°C |
| 30X | 30" at 94°C |
| | 1' at 55°C |
| | 1' at 68°C |
| 1X | 7 at 68°C |
| | hold at 4°C |

The following oligonucleotide sequencing primers were used to generate chain-termination fragments used for sequencing:
M13-40 biotin-GTTTTCCCAGTCACGACGTTGTA (SEQ ID NO:31)
M13007 biotin-TTCTGGCGTACCGTTCCTGTC (SEQ ID NO:32)

Sequencing reactions were performed using the DYEnamic™ ET Terminator Cycle Sequencing Kit with an ABI 9700 run in "Max Mode." Reactions were set up with the same volumes as follows:

### Forward Primers

| **Stocks** | **Vol per** Rx (uL) |
|---|---|
| Nuclease-free water | 6.5 |
| 10uM M13-40 | 0.5 |
| Sequencing reagent mastermix | 8 |
| Cleaned Up PCR Reaction | 5 |

### Reverse Primers

| **Stocks** | **Vol per** Rx (uL) |
|---|---|
| Nuclease-free water | 6.5 |
| 10uM M13007 | 0.5 |
| Sequencing reagent mastermix | 8 |
| Cleaned Up PCR Reaction | 5 |

Thermal cycling conditions for the DYEnamic™ ET Terminator Cycle Sequencing Kit used were as follows:

| | |
|---|---|
| 25X | 20" at 95°C |
| | 15" at 50°C |
| | 1' at 60°C |

The thermal cycling conditions for the BigDye® Terminator v3.1 Cycle Sequencing Kit were:

| | |
|---|---|
| 1X | 1' at 96°C |
| 25X | 10" at 96°C |
| | 5" at 50°C |
| | 4' at 60°C |

The resulting sequencing products were cleaned using magnetic streptavidin (MGSA).

The chimeric primers of the present invention produced normalization PCR sequencing templates capable of producing high quality sequence for two different regions of HIV, indicating that the primer design strategy is generally applicable and will not be limited to HIV applications. As expected, a defined region of the genome can be sequenced reliably and processed by clinical interpretation software.
## Claims

1. A method to prepare a template for sequencing the L10 codon of the HIV protease gag gene comprising the steps of:
providing a polynucleotide template having refractory region upstream of a target region to be sequenced;
hybridizing to the polynucleotide template an oligonucleotide forward primer of SEQ ID NO: 8,9, 11, 12 or 13 ;
initiating polymerase-catalyzed DNA synthesis, thereby producing a second replicate polynucleotide template comprising the above oligonucleotide forward primer, for use in a subsequent sequencing reaction.

2. An oligonucleotide forward primer comprising the sequence of any of SEQ ID NO: 8, 9,11,12,13.

## Patentansprüche

1. Verfahren zur Herstellung eines Templates zur Sequenzierung des L10 Codons des HIV Protease gag-Gens, umfassend die Schritte:
Bereitstellen eines Polynukleotid-Templates mit einer refraktären Region oberhalb einer zu sequenzierenden Zielregion;
Hybridisieren eines Oligonukleotid-Forward-Primers gemäß SEQ ID NO: 8, 9 11, 12 oder 13 mit dem Polynukleotid-Template;
Initiieren einer Polymerase-katalysierten DNA-Synthese, wodurch eine weitere Kopie des Polynukleotid-Templates, das den oben genannten Oligonukleotid-Forward-Primer enthält, produziert wird, zur Verwendung in einer nachfolgenden Sequenzierungsreaktion.

2. Oligonukleotid-Forward-Primer enthaltend die Sequenz von SEQ ID NO: 8, 9, 11, 12 oder 13.

## Revendications

1. Procédé pour préparer une matrice pour le séquençage du codon L10 du gène de la protéase gag du VIH, comprenant les étapes consistant à:
- fourniture d'une matrice polynucléotidique ayant réfractaire région en amont d'une région cible à séquencer;
- hybrider à la matrice polynucléotidique d'un oligonucléotide d'amorce directe de SEQ ID NO: 8, 9, 11, 12 ou 13;
- initier la synthèse d'ADN polymérase-dépendante, produisant ainsi une seconde matrice répliquée polynucléotidique comprenant l'oligonucléotide amorce sens ci-dessus,
pour une utilisation dans une réaction de séquençage ultérieur.

2. Un oligonucléotide amorce sens comprenant la séquence de l'une quelconque des SEQ ID NO: 8, 9,11,12,13.


<110> Siemens Medical Solutions Diagnostics Reynolds, Rebecca
<120> NOVEL OLIGONUCLEOTIDE PRIMERS AND METHODS FOR DNA REPLICATION
<130> 49493-337
<140> Not yet assigned
   <141> 2007-05-01
<150> 60/746,124
   <151> 2006-05-01
<160> 32
<170> PatentIn version 3.3
<210> 1
   <211> 23
   <212> DNA
   <213> Homo sapiens
<400> 1
   gttttcccag tcacgacgtt gta 23
<210> 2
   <211> 21
   <212> DNA
   <213> Homo sapiens
<400> 2
   ttctggcgta ccgttcctgt c 21
<210> 3
   <211> 15
   <212> DNA
   <213> Homo sapiens
<400> 3
   gttgagctga gaact 15
<210> 4
   <211> 30
   <212> DNA
   <213> Homo sapiens
<400> 4
   gttgagctga gaactcgaag acaagagctg 30
<210> 5
   <211> 40
   <212> DNA
   <213> Homo sapiens
<400> 5
   gttgagctga gaactggagt acgaccgaag acaagagctg 40
<210> 6
   <211> 40
   <212> DNA
   <213> Homo sapiens
<400> 6
   ctgctgcaga tagactcgaa caggtagtgt gaagtcgaca 40
<210> 7
   <211> 21
   <212> DNA
   <213> Homo sapiens
<400> 7
   ttccctcaga tcactctttg g 21
<210> 8
   <211> 74
   <212> DNA
   <213> Homo sapiens
<400> 8
   gttttcccag tcacgacgtt gtagttgagc tgagaactcg aagacaagag ctgttccctc 60
   agatcactct ttgg 74
<210> 9
   <211> 59
   <212> DNA
   <213> Homo sapiens
<400> 9
   gttttcccag tcacgacgtt gtagttgagc tgagaacttt ccctcagatc actctttgg 59
<210> 10
   <211> 44
   <212> DNA
   <213> Homo sapiens
<400> 10
   gttttcccag tcacgacgtt gtattccctc agatcactct ttgg 44
<210> 11
   <211> 72
   <212> DNA
   <213> Homo sapiens
<400> 11
   ttctggcgta ccgttcctgt cgttgagctg agaactcgaa gacaagagct gttccctcag 60
   atcactcttt gg 72
<210> 12
   <211> 82
   <212> DNA
   <213> Homo sapiens
<400> 12
   ttctggcgta ccgttcctgt cgttgagctg agaactggag tacgaccgaa gacaagagct 60
   gttccctcag atcactcttt gg 82
<210> 13
   <211> 82
   <212> DNA
   <213> Homo sapiens
<400> 13
   ttctggcgta ccgttcctgt cgttgagctg agaactggag tacgaccgaa gacaagagct      60
   gttccctcaa atcactcttt gg                                               82
<210> 14
   <211> 24
   <212> DNA
   <213> Homo sapiens
<400> 14
   gaatattgct ggtgatcctt tcca 24
<210> 15
   <211> 77
   <212> DNA
   <213> Homo sapiens
<400> 15
   gttttcccag tcacgacgtt gtagttgagc tgagaactcg aagacaagag ctggaatatt      60
   gctggtgatc ctttcca                                                     77
<210> 16
   <211> 62
   <212> DNA
   <213> Homo sapiens
<400> 16
   gttttcccag tcacgacgtt gtagttgagc tgagaactga atattgctgg tgatcctttc      60
   ca                                                                     62
<210> 17
   <211> 47
   <212> DNA
   <213> Homo sapiens
<400> 17
   gttttcccag tcacgacgtt gtagaatatt gctggtgatc ctttcca 47
<210> 18
   <211> 75
   <212> DNA
   <213> Homo sapiens
<400> 18
   ttctggcgta ccgttcctgt cgttgagctg agaactcgaa gacaagagct ggaatattgc 60
   tggtgatcct ttcca 75
<210> 19
   <211> 60
   <212> DNA
   <213> Homo sapiens
<400> 19
   ttctggcgta ccgttcctgt cgttgagctg agaactgaat attgctggtg atcctttcca 60
<210> 20
   <211> 45
   <212> DNA
   <213> Homo sapiens
<400> 20
   ttctggcgta ccgttcctgt cgaatattgc tggtgatcct ttcca 45
<210> 21
   <211> 77
   <212> DNA
   <213> Homo sapiens
<400> 21
   gttttcccag tcacgacgtt gtagttgagc tgagaactcg aagacaagag ctggaatatt 60
   gctggtgatc ctttcca 77
<210> 22
   <211> 87
   <212> DNA
   <213> Homo sapiens
<400> 22
   gttttcccag tcacgacgtt gtactgctgc agatagactc gaacaggtag tgtgaagtcg 60
   acagaatatt gctggtgatc ctttcca 87
<210> 23
   <211> 25
   <212> DNA
   <213> Homo sapiens
<400> 23
   gcctgaaaat ccatacaata ctcca 25
<210> 24
   <211> 76
   <212> DNA
   <213> Homo sapiens
<400> 24
   ttctggcgta ccgttcctgt cgttgagctg agaactcgaa gacaagagct ggcctgaaaa 60
   tccatacaat actcca 76
<210> 25
   <211> 86
   <212> DNA
   <213> Homo sapiens
<400> 25
   ttctggcgta ccgttcctgt cgttgagctg agaactggag tacgaccgaa gacaagagct 60
   ggcctgaaaa tccatacaat actcca 86
<210> 26
   <211> 81
   <212> DNA
   <213> Homo sapiens
<400> 26
   ttctggcgta ccgttcctgt cgttgagctg agaactcgaa gacaagagct gttagatgaa 60
   gacttcagga agtatactgc a 81
<210> 27
   <211> 22
   <212> DNA
   <213> Homo sapiens
<400> 27
   caaactccca ttcaggaatc ca 22
<210> 28
   <211> 75
   <212> DNA
   <213> Homo sapiens
<400> 28
   gttttcccag tcacgacgtt gtagttgagc tgagaactcg aagacaagag ctgcaaactc 60
   ccattcagga atcca 75
<210> 29
   <211> 85
   <212> DNA
   <213> Homo sapiens
<400> 29
   gttttcccag tcacgacgtt gtactgctgc agatagactc gaacaggtag tgtgaagtcg 60
   acacaaactc ccattcagga atcca 85
<210> 30
   <211> 85
   <212> DNA
   <213> Homo sapiens
<400> 30
   gttttcccag tcacgacgtt gtactgctgc agatagactc gaacaggtag tgtgaagtcg     60
   acacaaactc ccactcagga atcca                                           85
<210> 31
   <211> 23
   <212> DNA
   <213> Homo sapiens
<400> 31
   gttttcccag tcacgacgtt gta 23
<210> 32
   <211> 21
   <212> DNA
   <213> Homo sapiens
<400> 32
   ttctggcgta ccgttcctgt c 21